# FASCICULE DE BREVET EUROPEEN

(11) **EP 0 399 928 B1**
(45) Date de publication et mention de la délivrance du brevet: **23.06.1993**
(21) Numéro de dépôt: 90420240.5
(22) Date de dépôt: 17.05.1990
(51) Int. Cl.: F27B 14/10, F27B 14/08, H05B 6/22

(54) **Creuset froid à vidange par le fond**
Kalter Tiegel mit Bodenentleerung
Cold crucible with bottom emptying

(30) Priorité: 19.05.1989 FR 8907027
(43) Date de publication de la demande: 28.11.1990
(73) Titulaire: CEZUS Compagnie Européenne du Zirconium, F-92400 Courbevoie (FR)
(72) Inventeur: Garnier, Marcel, F-38410 Uriage (FR); Gleizes, Isabelle, F-38400 St. Martin d'Heres (FR); Paillere, Patrick, D-73400 Ugine (FR); Vernay,Pierre, F-38240 Meylan (FR)
(74) Mandataire: Vanlaer, Marcel

(56) Documents cités:
- EP-A- 0 150 484
- FR-A- 2 052 082
- US-A- 3 702 368
- US-A- 4 403 955

## Description

La présente invention est relative à un creuset froid à vidange par le fond.

L'élaboration de matériaux à très haut degré de pureté, de matériaux réactifs et de matériaux à très haut point de fusion nécessite généralement leur passage par l'état liquide et le maintien dans cet état pendant un temps suffisamment long pour obtenir l'homogénéisation du liquide vis à vis des divers constituants ou de la température ou encore pour permettre à des réactions chimiques de s'accomplir au sein du liquide. Pour ce faire, il importe qu'un brassage turbulent anime le liquide et que celui-ci ne puisse réagir sur aucune des parois matérielles généralement indispensables pour contenir le liquide.

La solution mise en oeuvre pour réaliser cette élaboration consiste le plus souvent à utiliser un creuset froid à chauffage par induction.
Un tel creuset est formé par une paroi conductrice, généralement en cuivre, constituée de plusieurs secteurs longitudinaux, creux, isolés électriquement les uns des autres et parcourus intérieurement par une circulation de fluide réfrigérant qui permet de maintenir leur surface en contact avec le métal liquide à une température généralement voisine ou inférieure à 300°C. Ce creuset est placé à l'intérieur d'un inducteur alimenté en courant alternatif qui crée des courants induits dans les secteurs, courants qui se referment en parcourant la paroi interne du creuset et dans lequel ils créent un champ magnétique.

Tout matériau électroconducteur placé dans un tel creuset est le siège de courants induits qui permettent de le chauffer jusqu'à la fusion tout en le brassant.

Ces creusets peuvent fonctionner de deux manières :
- d'une part en lévitation. En effet, les parois internes du creuset sont généralement coniques vers le bas de sorte que la section de ce dernier est plus petite en bas qu'en haut. Or, par principe, le produit du champ magnétique par la section du creuset est sensiblement constant sur l'axe du creuset. Il en résulte donc un champ magnétique qui croît fortement depuis le haut jusqu'en bas du creuset. Cette configuration est parfaitement adaptée à la lévitation puisque les forces de répulsion induites dans un matériau électroconducteur placé dans le creuset sont très fortes à la partie inférieure du matériau et décroissent vers la partie supérieure. Un liquide peut donc être maintenu en lévitation stable dans un tel creuset.
   Le creuset étant froid, toute interruption de puissance est sans aucun danger car le liquide se répandant dans le creuset, véritable lingotière, se solidifie. La remise sous tension de l'inducteur permet de refondre le matériau et de reprendre la lévitation.
   En état de lévitation, le matériau qui peut être placé au contact d'une atmosphère contrôlée ne subit aucune dégradation. De plus, les forces électromagnétiques induisent un brassage turbulent dans le liquide. Les conditions d'élaboration de manière à obtenir une haute pureté peuvent donc être réalisées;
- d'autre part en auto-creuset. En effet, dans ce même type de creuset, lorsque la masse de matériau liquide devient trop importante, les forces électromagnétiques ne peuvent plus équilibrer les forces de pesanteur et le contact entre le matériau et la paroi du creuset devient inévitable. Cependant, aucune dégradation du matériau n'apparaît car lorsqu'il vient à l'état liquide au contact de la paroi, une solidification locale se produit. Le matériau liquide est alors contenu dans une paroi formée par la pellicule solide constituée par le matériau lui-même. De plus, le contact entre le matériau solide et le creuset est froid : il n'y a donc ni pollution, ni réaction. L'élaboration sous haute pureté du matériau liquide brassé par le champ magnétique est donc également possible.

Dans les deux cas, il n'existe aucune limite de température. Dans la lévitation, l'élaboration se fait sans contact et les échanges thermiques entre le matériau et le creuset se font par rayonnement : ils restent donc très limités.

Dans l'auto-creuset, il suffit de prévoir un refroidissement énergique des secteurs du creuset pour maintenir une croûte de matériau solide sur la paroi interne.

Même avec des températures de charge voisines de 3000°C, l'eau suffit comme fluide de refroidissement du creuset.

Le matériau ayant été élaboré dans lesdits creusets, il est alors nécessaire de le récupérer. Cette opération de récupération doit être réalisée dans des conditions telles qu'elles ne provoquent aucune pollution afin de ne pas perdre les avantages obtenus au cours de l'élaboration. Il faut aussi que cette récupération soit rapide, commode et complète.

Plusieurs modes de récupération ont été proposés :

Ainsi, on peut, de façon très élémentaire, laisser le matériau se refroidir dans le creuset et le démouler; mais, cette opération n'est pas très commode et ne permet pas d'obtenir un produit de forme déterminée : on est donc ensuite obligé de le refondre.

Dans le brevet US 4738713, le matériau liquide est versé du creuset dans un moule. Comme le creuset ne possède qu'une seule ouverture placée à la partie supérieure, la récupération ne peut avoir lieu que par basculement. A moins de disposer d'un matériel sophistiqué permettant d'isoler le creuset et le moule de l'atmosphère, cette opération est généralement réalisée dans l'air et entraîne inévitablement une pollution du matériau par oxydation ou nitruration. En outre, le basculement n'est pas commode car il nécessite, pour éviter une désolidarisation du creuset de ses amenées d'eau de refroidissement et de courant électrique, de recourir à un système mécanique très compliqué.

On peut encore envisager comme dans le cas du brevet français 2561761 d'équiper le creuset d'une goulotte d'évacuation latérale, mais cela a l'inconvénient d'une part de nécessiter des modifications de secteurs pour assurer son passage, d'autre part de ne pas permettre la vidange totale du creuset. En outre, avec les matériaux à haut point de fusion, cette goulotte risque de se boucher facilement si elle n'est pas équipée de moyens de chauffage convenables.

C'est pourquoi la demanderesse consciente des difficultés que pouvaient présenter ces méthodes de récupération a cherché et trouvé un moyen de vidange qui n'entraîne pas de pollution et qui soit à la fois commode, complet et rapide.

D'où l'invention consistant en un creuset froid à vidange par le fond formé par une paroi constituée de plusieurs secteurs longitudinaux, creux, isolés électriquement au moins partiellement les uns des autres, parcourus intérieurement par un fluide réfrigérant et destinés à être placés à l'intérieur d'un inducteur et dans lequel ladite paroi est munie à sa partie inférieure d'une ouverture, ladite invention étant caractérisée en ce qu'à l'intérieur de ladite ouverture est placé un obturateur amovible, sectorisé et refroidi.

Ainsi, l'invention consiste en somme à placer dans l'ouverture inférieure du creuset un obturateur conçu comme un creuset de dimensions réduites, c'est-à-dire présentant des secteurs creux isolés électriquement les uns des autres et parcourus intérieurement par un fluide réfrigérant. Ces secteurs ne s'allongent pas sur toute la section horizontale de l'obturateur et laissent place à une ouverture centrale d'un diamètre généralement inférieur à 3 mm, ouverture qui peut être éventuellement fermée avec un matériau isolant de l'électricité.

Lorsque le courant circule dans l'inducteur, les courants induits apparaissent dans le creuset. A la partie inférieure du creuset, ces courants induits créent un champ magnétique qui est à l'origine de nouveaux courants induits dans l'obturateur placé dans l'ouverture. En position "fermée", il n'y a donc aucun trou matériel ni aucun trou magnétique à la partie basse du creuset qui se comporte comme un creuset constitué d'une seule pièce. La lévitation est donc possible.

Pour permettre la coulée du matériau liquide maintenu en lévitation, il suffit d'éliminer rapidement le petit creuset fermant le creuset principal. Le champ magnétique présente alors un défaut sur l'axe à la partie inférieure du liquide qu'il ne peut plus supporter. La coulée se produit alors par l'ouverture ainsi créée.

Dans le cas de l'auto-creuset, la procédure est la même à la différence près que l'ablation du petit creuset crée un trou magnétique mais la croûte de métal solidifiée empêche le liquide de s'écouler. Deux possibilités existent alors pour libérer le trou matériel :
- soit une augmentation de puissance dans l'inducteur qui se traduit par deux effets : d'une part, une augmentation de la température du liquide qui s'accompagne d'une augmentation de l'intensité de brassage, conditions propices à la refusion de la croûte solide dans la zone où la paroi du creuset refroidi n'évacue plus les calories; d'autre part, les bords de l'orifice se comportent comme un concentreur de champ magnétique qui, lors d'une augmentation de puissance, accroît localement l'effet Joule développé dans le matériau et favorise sa fusion;
- soit l'utilisation d'un inducteur annexe placé à la base du creuset principal conçu à cet effet et dont la mise sous tension entraîne la fusion de la croûte solide fermant l'orifice et permet la coulée.

De préférence, le nombre de secteurs de l'obturateur est inférieur ou égal au nombre de secteurs du creuset et en tout cas au moins égal à deux.
De plus, il est préférable de donner aux parois de l'ouverture et de l'obturateur une forme conique vers le haut afin de faciliter le dégagement de l'obturateur.
Ce dégagement est réalisé par tout moyen mécanique relié à l'obturateur et permettant d'obtenir successivement un mouvement vertical de haut en bas puis un mouvement de translation à droite ou à gauche de manière à ne laisser aucun obstacle sur le passage du matériau qui s'écoule.
Un tel obturateur peut être utilisé pour un grand nombre d'opérations d'élaboration.

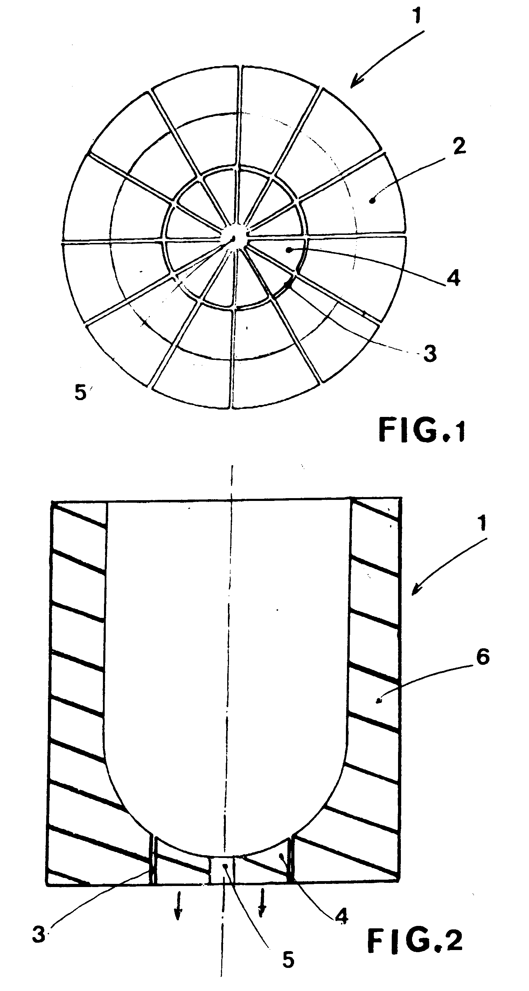

L'invention sera mieux comprise à l'aide de la planche de dessins ci-jointe qui représente :
. Fig. 1 une vue de dessus d'un creuset selon l'invention
. Fig. 2 une vue en coupe axiale verticale du même creuset.

De façon plus détaillée, on distingue sur la figure 1 le creuset 1 partagé en douze secteurs 2 creux et refroidis entourant une ouverture 3 dans laquelle est placé un obturateur 4 composé également de douze secteurs creux et refroidis qui n'occupent pas toute la section de l'ouverture et laissent place à un trou circulaire 5.

Sur la figure 2, le creuset 1 à paroi latérale 6 muni en son fond et suivant son axe vertical d'une ouverture 3 dans laquelle est placé l'obturateur amovible composé des secteurs 2 creux et refroidis délimités vers le centre par le trou. Ce creuset est entouré d'un inducteur et les secteurs sont refroidis par des circuits d'eau; ces éléments ne sont pas représentés.

En fonctionnement, les éléments du matériau à élaborer ayant été introduits dans le creuset, on alimente l'inducteur en courant alternatif de manière à fondre lesdits matériaux, à les brasser et éventuellement à les faire réagir. A la fin de la réaction, le matériau étant homogène et complètement fondu, on déplace l'obturateur rapidement: d'abord verticalement, puis latéralement de manière à laisser s'écouler le matériau.

L'écoulement du matériau peut être réglé en jouant sur l'intensité du courant qui alimente l'inducteur.
En effet, si on maintient une intensité très élevée, le matériau peut rester en lévitation; par contre, si on abaisse progressivement cette intensité, on supprime progressivement la lévitation et augmente le débit coulé de façon régulière.

L'invention peut être illustrée à l'aide des exemples d'application suivants :

### Exemple 1

Un creuset de diamètre 100 mm formé de 12 secteurs longitudinaux, creux, isolés électriquement les uns des autres, parcourus intérieurement par un fluide réfrigérant et placés à l'intérieur d'un inducteur alimenté en courant alternatif de fréquence 15 kHz et de puissance 80 kW et dont le fond est muni d'une ouverture de diamètre 16 mm, a été équipé d'un obturateur de diamètre voisin de celui de l'ouverture, de hauteur 40 mm et formé de 4 secteurs isolés et refroidis.

Dans ce creuset, on a fondu 400 g de nickel qui ont été vidangés ensuite par le fond en 3 secondes en imprimant rapidement à l'obturateur d'abord un mouvement vertical de haut en bas d'ampleur suffisante pour le dégager de l'ouverture, puis un mouvement latéral convenable pour ne pas gêner l'écoulement du matériau. Le produit obtenu ne présentait aucune pollution.

### Exemple 2

Un creuset de diamètre 60 mm formé de 8 secteurs isolés, creux et refroidis placés à l'intérieur d'un inducteur alimenté en courant alternatif de fréquence 12 kHz et de puissance 60 kW et dont le fond est muni d'une ouverture de diamètre 8 mm a été équipé d'un obturateur de diamètre voisin de celui de l'ouverture, de hauteur 30 mm et formé de 2 secteurs isolés et refroidis.
Dans ce creuset, on a fondu 150 g d'un mélange de titane et d'aluminium en proportions telles qu'elles conduisent à la formation de l'alliage Ti₃Al. Ledit alliage a été vidangé ensuite par le fond en une seconde en procédant aux mêmes manoeuvres que dans l'Exemple 1.
Le produit obtenu ne présentait aucune trace de pollution.

L'invention trouve son application dans l'élaboration de matériaux de grande pureté récupérables à l'état liquide de façon commode, complète et rapide.

## Revendications

1. Creuset à vidange par le fond formé par une paroi (6) conductrice constituée de plusieurs secteurs longitudinaux, creux, isolés électriquement au moins partiellement les uns des autres, parcourus intérieurement par un fluide réfrigérant et destinés à être placés à l'intérieur d'un inducteur et dans lequel ladite paroi est munie à sa partie inférieure d'une ouverture (3) caractérisé en ce qu'à l'intérieur de ladite ouverture est placé un obturateur (4) amovible, sectorisé et refroidi.

2. Creuset selon la revendication 1 caractérisé en ce que le nombre de secteurs de l'obturateur est inférieur ou égal au nombre de secteurs du creuset.

3. Creuset selon la revendication 1 caractérisé en ce que les parois de l'obturateur et de l'ouverture sont coniques vers le haut.

## Patentansprüche

1. Tiegel zur Leerung durch den Boden, der durch eine leitende Wand (6) gebildet ist, die aus mehreren hohlen, wenigstens teilweise untereinander elektrisch isolierten Längssektoren besteht, die innerlich von einem Kühlfluid durchströmt werden und zur Anordnung im Inneren eines Induktors bestimmt sind, und in dem diese Wand an ihrem unteren Teil mit einer Öffnung (3) versehen ist,
**dadurch gekennzeichnet,**
daß im Inneren dieser Öffnung ein herausnehmbares, sektorunterteiltes und gekühltes Verschlußstück (4) angeordnet ist.

2. Tiegel nach dem Anspruch 1,
**dadurch gekennzeichnet,**
daß die Sektorenzahl des Verschlußstücks kleiner als die oder gleich der Sektorenzahl des Tiegels ist.

3. Tiegel nach dem Anspruch 1,
**dadurch gekennzeichnet,**
daß die Wände des Verschlußstücks und der Öffnung nach oben konisch sind.

## Claims

1. A bottom discharge crucible formed by a conductive wall (6) consisting of a plurality of longitudinal hollow segments which are at least partially insulated electrically from one another, traversed internally by a cooling fluid and deemed to be disposed inside an inductor and in which the said wall is provided in its bottom part with an aperture (3) characterised in that inside the said aperture is placed a removable segmental and cooled occluding means (4).

2. A crucible according to Claim 1, characterised in that the number of segments in the occluding means is less than or equal to the number of segments in the crucible.

3. A crucible according to Claim 1, characterised in that the walls of the occluding means and of the aperture are outwardly conical.
